# EUROPEAN PATENT APPLICATION

(11) **EP 2 529 728 A1**
(43) Date of publication of application: **05.12.2012**
(21) Application number: 11734269.1
(22) Date of filing: 19.01.2011
(51) Int. Cl.: A61K 9/08, A61K 31/165, A61P 31/04, A61K 47/22, A61K 47/10

(54) **PHARMACEUTICAL COMPOSITIONS FOR THE TREATMENT OF BACTERIAL INFECTIONS**

(30) Priority: 21.01.2010 BR PI1002021
(71) Applicant: Nanocore Biotecnologia S.A., Campinas - SP, Cep: 13069-380 (BR)
(72) Inventor: RODRIGUES JR., José Marciel, Cep: 13107-110 (BR); LIMA, Karla de Melo, Cep: 13107-110 (BR)
(74) Representative: Fritz, Edmund Lothar
(86) International application number: PCT/BR2011/000012
(87) International publication number: WO 2011/088532

(57) **Abstract**

The present invention is related to pharmaceutical compositions capable of inhibiting or eliminating an infectious process caused by bacteria in warm-blooded animals. The present invention describes a stable formulation with a minimized methylpyrrolidone concentration. In order to reduce N-methylpyrrolidone concentration, a mixture was developed that eliminates the need to add preservatives and exhibiting a suitable viscosity and ensures the solubility of the composition when administered in drinking water at a ratio of dilution of up to 5,000 times in chlorinated or non-chlorinated water.

## Description

### Field of the Invention

The present invention relates to pharmaceutical compositions capable of inhibiting or eliminating an infection caused by bacteria in warm-blooded animals. The invention relates to formulations containing antibiotics composition to be administered by different routes for the treatment of bacterial infections in warm-blooded animals, including oral and intravenous routes.

### Background of the Invention

The use of antibiotics in food animals is of great importance to prevent and treat different types of infection in warm-blooded animals, whether cattle, goats, sheep, poultry and pigs, and also for the treatment of pets as cats, dogs and horses. The use of antibiotic therapy becomes even more important in cases of infections for which there are no prophylactic methods.

In practice, these products can be administered directly to individuals through pharmaceutical forms or can be added to food or drinking water, especially in the production of poultry and pork. The use of this practice has been increasingly important for animals in confinement where the incidence of respiratory disease is more common.

The costs of production losses in consequence of respiratory diseases are important and it is estimated that the losses exceeds US$ 1 billion annually in the United States only in cattle. In Europe it ranges from 75 to 120 million U.S. dollars considering the different countries. Moreover, it is reported the risk of contamination between species and may even infect humans, in need of an appropriate antibiotics therapy and particularly a means of effective administration thus avoiding high doses which can lead to toxic or under dosing, which can generate resistant strains.

The production losses are not only related to morbidity and mortality, but also to reduced productivity of meat and milk and spending on treatment.

The main causative agents of respiratory infections in these species of production are of the genera Pasteurella, Haemophilus, Streptococcus, Bordetella, Salmonella, Actinobacillus and also Mycoplasma.

The most commonly used chemotherapeutic agents are intended to combat the infection which, among other symptoms, resulting in local and systemic inflammation, damage to lung tissue, fever and general morbidity with reduced immune response and, consequently, association with other infections especially from fungal origin thus making the introduction of antibiotic treatment a priority.

Among the antibiotics used in veterinary medicine are the aminocyclitols (spectinomycin to apramycin), aminoglycosides (gentamicin and neomycin), beta-lactams (penicillins, amoxicillin, ceftiofur and cefarin), fluoroquinolones (enrofloxacin, ciprofloxacin and danofloxacin), the lincosamides (lincomycin, clindamycin and pirlimycin), macrolides (erythromycin, tilmicosin, tylosin), sulfonamides (combined or not), tetracyclines (tetracycline, chlortetracycline and oxytetracycline) and amphenicols (florfenicol and thiamphenicol).

The latter interferes with protein synthesis and gain more and more interest in medical practice because they are effective against gram negative and gram positive bacteria which cause respiratory infections. Because they are insoluble in water in usual concentrations, few formulations ready for use are available and there is no formulation available in major veterinary market in Brazil, intended for dilution in drinking water that would be useful, especially for poultry and pigs.

The state of the art shows a patent claiming a composition containing 10-50% of florfenicol in a solvent containing a derivate of pyrrolidone, a viscosity reducing agent and polyethylene glycols. (U.S. Patent 5,082,863).

The patent application WO 2004/110494 A1 claims a formulation containing 10-50% of florfenicol in a carrier which must necessarily contain triacetin, dimethylacetamide, or combination of these two solvents with pyrrolidone. The formulation has a viscosity suitable for syringeability, but the constituents of this carrier are marketed at high costs.

The patent application WO 2003/028648 describes a formulation containing florfenicol dissolved in N-methylpyrrolidone (concentrations above 40%) containing preservatives, including, ethanol in the proportion of 5 to 100 mg/mL (0.5 to 10% v/v). This formulation showed low viscosity, even at low temperatures, proving to be viable for the injectable route as it presents good conditions of syringeability. Importantly, the authors suggest ethanol at a concentration of 0.5 to 10% v/v which corresponds to a concentration which would correspond to about 80% of N-methyl pyrrolidone. In normal use doses of the product for the treatment of infections for which is indicated by injectable route in pigs and bovines, it corresponds to about 27-54 mg/kg of methylpyrrolidone. Although it is a relatively well tolerated product, there is no literature data to ensure safety in this higher dose which may be considered high. In this formulation the role of the ethanol is as a preservative and is indicated only by injectable route.

The patent FR 07 09197 claims a formulation of water-soluble florfenicol in the presence of surfactants. The florfenicol is dissolved in a formulation containing 50% methylpyrrolidone in the presence of surfactants and polyethylene glycol.

The patent application WO 2006/067138 discloses a low viscosity formulation based on a composition containing N-methyl pyrrolidone as carrier and necessarily an ether of 1,2-ethanediol oligo- or its polymers (preferably diethyl glycol monoethyl ether).

In the pharmaceutical market it is found formulations available in a concentration of 30% with high viscosity which makes difficult the handling of the animals at the moment of injectable application by having poor conditions of syringeability. These formulations are not recommended for dilution in drinking water which makes difficult their use in poultry species whose injectable route is not desirable.

Other products offer a mixture of florfenicol as a pre-mixture (PREMIX) in feed for poultry and pork. The mixing process is complex, resulting in products not necessarily homogeneous and of unwieldy which could be solved, for example if there is a formulation to be diluted in the drinking water of animals.

Thus, the search for a product with a reduced concentration of N-methylpyrrolidone, without use of additives such as preservatives and without surfactants, with an appropriate viscosity and which possess solubility after administration, it is still a need in the art.

### Summary of the Invention

In the present invention it is described a stable formulation whose concentration of methylpyrrolidone is equal to or less than 38% v/v (39.1 w/v) allowing, at indicated dose, the maximum administration which is about 41 mg/kg which has been described as acceptable dose for the proposed formulation (EMEA/MRL/615/99-FINAL-REV May 1, 2008). In order to reduce the concentration of N-methylpyrrolidone it was developed a carrier containing ethanol and glycerin, low cost solvents commonly used in formulations for enteral and parenteral use, including orally or injection, with long history of safety in humans and animals, which eliminates the need to add preservatives and exhibiting a viscosity suitable to ensure solubility after administration in drinking water at a ratio of up to 5,000 times dilution in water chlorinated or not chlorinated with no added surfactants.

The present invention describes formulations of low viscosity consisting of a safe solvent from the standpoint of administration route and target species, the formulations being free of polymer, free of triacetin and free of dimethylacetamide, containing amphenicols, preferably florfenicol, and thiamphenicol, which can be administered by enteral and parenteral routes, preferably through injection or orally by addition to drinking water. The product has a low viscosity and can be diluted into drinking water, chlorinated or not chlorinated, without precipitation of the drug.

### Detailed Description of Invention

In the present invention it were developed stable pharmaceutical compositions containing antibiotics for the treatment of infections in warm-blooded animals, preferably for the treatment of respiratory infections in livestock animals. The pharmaceutical compositions are to be added to drinking water or by injectable route depending on the manufacturing process. In the latter case the manufacturing process must include a sterilizing filtration.

The pharmaceutical composition for the treatment of infections in warm-blooded animals of the invention comprises:
(a) at least one drug selected from the group of antibiotics amphenicols, or amphenicols mixture;
(b) at least one carrier selected from the group of pyrrolidones in a concentration equal to or less than 38% v/v;
(c) a second carrier which is glycerin; and
(d) a third solvent which is ethanol,

the mixture above resulting in a product with low viscosity.

In a more preferred embodiment, the pharmaceutical compositions of this invention are free of surfactants and contain:
(a) at least one antibiotic of the class of amphenicols (Formula 1), preferably florfenicol (Formula 2) and thiamphenicol, in concentrations ranging from 1-50%, preferably at a concentration of 10% for dilution in drinking water and 30% for injectable route;
(b) a carrier of N-methylpyrrolidone in the concentration ranging from 20-38% v/v, preferably between 30 and 38% v/v);
(c) a second carrier consisting of glycerin in the concentration of 15-50% v/v preferably between 20 and 40% v/v; and
(d) a third carrier consisting of ethanol at a concentration of between 12 and 50% v/v.

The composition has low viscosity of about 12 cps for a 10% formulation and up to 30 cps for a 30% formulation at 25°C. This formulation is readily filtered on sterilizing filters of 0.2 micrometers and can be administered by injectable route, preferably in a concentration of 30%, or in drinking water, preferably at a concentration of 10%.

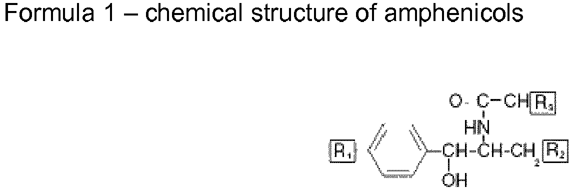

| | R₁ | R₂ | R₃ |
|---|---|---|---|
| Chloramphenicol | - NO₂ | OH | = Cl₂ |
| Azidamfenicol | - NO₂ | - OH | 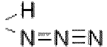 |
| Thiamphenicol | - SO₂CH₃ | -OH | = Cl₂ |
| Fforfenicol | - SO₂CH₃ | - F | = Cl₂ |

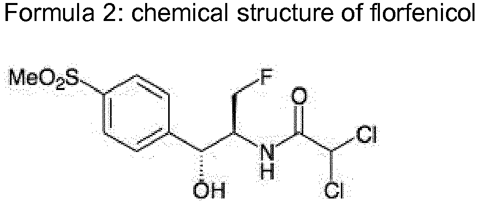

The invention object of this patent application can be best described by the following examples, which should not be considered as limiting the scope of protection.

### Example 1:

| **Constituents** | **Concentration (100 mL)** |
|---|---|
| Florfenicol | 10,00g |
| N-methy-2-pyrrolidone | 38 mL |
| Glycerin | 30 mL |
| Ethanol q.s.p. | 100 v/v (about 20 mL) |

The constituents are mixed in a single or multiple steps. Preferably, florfenicol was added to 30 mL of methylpyrrolidone and then ethanol and glycerin were added to under stirring. The final product, if desired, can be filtered and, then, it was packed in glass or plastic vials. Florfenicol can be optionally added to 30 mL of N-methylpyrrolidone. In this case it takes about 28 mL of ethanol to make up to volume.

### Example 2:

| **Constituents** | **Concentration (100 mL)** |
|---|---|
| Florfenicol | 30,00g |
| N-methy-2-pyrrolidone | 38 mL |
| Glycerin | 30 mL |
| Ethanol q.s.p. | 100 v/v (about 20 mL) |

The florfenicol was added to 30 mL of methyl pyrrolidone and, then, ethanol and glycerin were added under stirring. Florfenicol can be optionally added to 30 mL of N-methylpyrrolidone. In this case it takes about 28 mL of ethanol to make up to volume.

### Example 3:

| **Constituent** | **Concentration (100 mL)** |
|---|---|
| Thiamphenicol | 11,00g |
| N-methy-2-pyrrolidone | 38 mL |
| Glycerin | 30 mL |
| Ethanol q.s.p. | 100 v/v (about 20 mL) |

Thiamphenicol was added to 30 mL of methylpyrrolidone and then ethanol and glycerin were added under stirring. Florfenicol can be optionally added to 30 mL of N-methylpyrrolidone. In this case it takes about 28 mL of ethanol to make up to volume.

### Example 4:

| Constituinte | Concentração (100 mL) |
|---|---|
| Tianfenicol | 30,00g |
| N - metil - 2 - pirrolidona | 38 mL |
| Glicerina | 30 mL |
| Etanol q.s.p. | 100 v/v (cerca de 20mL) |

Thiamphenicol was added to 35 ml of methyl pyrrolidone and then were added to ethanol and glycerin with stirring. The product can be filtered on sterilizing filters. Florfenicol can be optionally added to 30 ml of N-methylpyrrolidone. In this case it takes about 28ml of ethanol to make up to volume.

### Example 5: Viscosity

Determination of viscosity was performed at 25°C using a Brookfield DV-I+ viscometer (Brookfield Engineering). The spindle used was number S31. The viscosity values found in these conditions were lower than 30 cps and ranged from 15 to 28.5 cps for the formulation of 30% florfenicol and lower than 12 cps ranging between 5 and 11.5 for the formulation containing 10% of florfenicol.

### Example 6: Stability

The samples obtained as described in Example 2, were stored in glass vials in a climatic chamber LCD 420 (Nova Etica), placed at temperatures of 30±2°C and 65±5% of relative humidity (RH), 40±2°C and 75±5% of RH and 50±2°C and 90±5% of RH.

It was evaluated the appearance of the product by visual inspection to identify, especially, any precipitation. Then, the determination of the contents of active ingredients was carried out. The determination of the content of the active ingredients in the product was carried out by high performance liquid chromatography (HPLC), following previously validated conditions.

The results found for the study of stability of the 10% Florfenicol formulation 10% are summarized in Tables 1-3.

Tables 4 and 5 show the results of the study of stability related to the period of use after dilution into drinking water, chlorinated and non-chlorinated, at a ratio of a part of the formulation to 5,000 parts of water.

The results of the initial analysis are within the limits of compliance showing that the product meets the parameters set (95-105%).

**Table 1 - Results from the analysis of the Formulation of Florfenicol 10% before and after storage at 30±2°C and 65±5% of RH.**

| Analysis | Initial | 3 months | 6 months |
|---|---|---|---|
| Aspect | Comply | Comply | Comply |
| Conc. Florfenicol | 9,679g/100mL (96,79% VR) | 9,921 g/100mL (99,21 %VR) | 9,964g/100mL (99,649% VR) |

**Table 2 - Results from the analysis of the Formulation of Florfenicol 10% before and after storage at 40±2°C and 75±5% RH.**

| Analysis | Initial | 1 month | 2 months | 3 months | 6 months |
|---|---|---|---|---|---|
| Aspect | Comply | Comply | Comply | Comply | Comply |
| Conc. Florfenicol | 9,679g/100mL (96,799% VR) | 9,679g/100mL (96,79% VR) | 9,948g/100mL (99,482% VR) | 9,485g/100mL (94,859% VR) | 9,671 g/100mL (96,714% VR) |

**Table 3 - Results from the analysis of the Formulation of Florfenicol 10% before and after storage at 50±2°C and 90±5% RH.**

| Analysis | Initial | 1 month | 2 months | 3 months |
|---|---|---|---|---|
| Aspect | Comply | Comply | Comply | Comply |
| Conc. Florfenicol | 9,679g/100mL (96,799% VR) | 9,931 g/100mL (99,318% VR) | 9,333g/100mL (93,333% VR) | 10,063g/100mL (100,636% VR) |

**Table 4 - Results from the analysis related to the period of use of the Formulation of Florfenicol 10% with chlorinated water before and after storage at 30±2°C and 65±5% RH.**

| Analysis | Initial | 20 days | 30 days |
|---|---|---|---|
| Aspect | Comply | Comply | Comply |
| Conc. Florfenicol | 9,448g/100mL (94,485% VR) | 9,791 g/100mL (97,914% VR) | 9,895g/100mL (98,955% VR) |

**Table 5 - Results from the analysis related to the period of use of the Formulation of Florfenicol 10% with non-chlorinated water before and after storage at 30±2°C and 65±5% RH.**

| Analysis | Initial | 20 days | 30 days |
|---|---|---|---|
| Aspect | Comply | Comply | Comply |
| Conc. Florfenicol | 9,371g/100mL (93,710% VR) | 9,688g/100mL (96,888% VR) | 9,771g/100mL (97,719% VR) |

The initial content found for florfenicol was 9.679g/100mL and for the stability study of the period of use was 9.448 g/100mL for the formulation in chlorinated water, and 9.371 g/100mL for formulation in non-chlorinated water. As can be seen in Tables 1-5, the developed product meets all the requirements of stability during the study period with a variation in content of actives of less than 5% in relation to the initial content when stored at 30°C and 65 % RH, 40°C and 75% RH and 50°C and 90% RH. The other analyzes show that the product remains within specification criteria throughout the study period.

The above description of the present invention was presented for the purpose of illustration and description. Consequently, variations and modifications consistent with the above teachings and the skill or knowledge of the relevant art are within the scope of the invention.

## Claims

1. A pharmaceutical composition for the treatment of infections in warm-blooded animals comprising:
(a) at least one drug selected from the group of antibiotics amphenicols, or amphenicols mixture;
(b) at least one carrier selected from the group of pyrrolidones in a concentration less than 40%;
(c) a second carrier containing glycerin; and
(d) a third carrier containing ethanol,
the mixture above resulting in a product with low viscosity, such as less than 50 cps at a temperature of 25°C.

2. The composition of claim 1 wherein the drug from the group of amphenicol is the florfenicol.

3. The composition of claim 1 wherein the drug from the group of amphenicol is the thiamphenicol.

4. The composition of claim 2 wherein the florfenicol is dissolved in a concentration from 1% to 50%, preferably from 10% to 30%.

5. The composition of claim 3 wherein thiamphenicol is dissolved in a concentration from 1% to 50%, preferably from 10% to 30%.

6. The composition of claim 1 wherein the compound the group of pyrrolidones is N-methyl-2-pyrrolidone.

7. The composition of claim 6 wherein the concentration of N-methyl-2-pyrrolidone is lower than 40% v/v, preferably between 20% to 38% v/v..

8. The composition of claim 1 wherein the concentration of glycerin is about 10% to 50%, preferably in the concentration of 30% v/v.

9. The composition of claim 1 wherein the concentration of ethanol is about 12% to 50% v/v, preferably in the concentration of about 12% to 30% w/v.

10. The composition according to claim 4 or 5 wherein said composition is administrated by injectable route in warm-blooded animals, preferably in the concentration of 30%.

11. The composition according to claim 4 or 5 wherein said composition is administrated by adding drinking water in warm-blooded animals, preferably in the concentration of 10% in relation to florfenicol and 11 % for thiamphenicol.

12. The composition according to claim 11 wherein said composition is administrated through drinking water, wherein no precipitation occurs during addition of drinking water and at least for the next 24 hours.

13. The composition according to claim 11 wherein said composition is diluted to at least 5,000 parts of water with no precipitation.

14. The composition according to claim 1 wherein the viscosity of said composition is less than 50 cps at a temperature of 25°C.
